# EUROPEAN PATENT APPLICATION

(11) **EP 2 940 126 A1**
(43) Date of publication of application: **04.11.2015**
(21) Application number: 13869392.4
(22) Date of filing: 27.12.2013
(51) Int. Cl.: C12N 5/071, A61L 27/00, A61P 27/02

(54) **HUMAN CORNEAL ENDOTHELIAL CELL SHEET**

(30) Priority: 27.12.2012 JP 2012286050
(71) Applicant: Nitta Gelatin Inc., Osaka 556-0022 (JP); Cornea Regeneration Institute Co., Ltd., Hyogo 657-0014 (JP)
(72) Inventor: YAMAGAMI, Satoru, Tokyo 113-0023 (JP); YAMAGUCHI, Masahiro, Tokyo 106-0047 (JP); SHIMA, Nobuyuki, Kobe-shi Hyogo 657-0014 (JP); YAMAUCHI, Miwa, Kobe-shi Hyogo 652-0811 (JP); HIRAOKA, Yosuke, Yao-shi Osaka 581-0024 (JP)
(74) Representative: Vossius & Partner Patentanwälte Rechtsanwälte mbB
(86) International application number: PCT/JP2013/085262
(87) International publication number: WO 2014/104366

(57) **Abstract**

The present invention provides a human corneal endothelial cell sheet having a curvature fitting a human corneal endothelial surface, particularly, the human corneal endothelial cell sheet supported by a cell support having a curvature fitting a human corneal endothelial surface, and a production method of a human corneal endothelial cell sheet having a curvature fitting a human corneal endothelial surface, which includes the following steps:
(1) a step of gelling a gelatin aqueous solution poured into a template having a curvature fitting a human corneal endothelial surface;
(2) a step of forming a sheet-like gelatin by drying the gelled gelatin obtained in (1);
(3) a step of obtaining a gelatin sheet by thermally crosslinking the sheet-like gelatin obtained in (2);
(4) a step of seeding human corneal endothelial cells on the gelatin sheet obtained in (3) to form a human corneal endothelial cell layer.

## Description

### Technical Field

The present invention relates to the development of a human corneal endothelial cell sheet and an effective corneal endothelial cell transplantation therapy using same.

### Background Art

Corneal endothelium consists of a single layer of cells constituting the innermost layer of cornea, and plays the most important role in the maintenance of the transparency of cornea, namely, eyesight by its barrier function and pumping function. Corneal endothelial cells do not grow and are not repaired in vivo, and the cell number decreases when they are damaged by trauma in surgery and the like or due to aging. Bullous keratopathy is a disease wherein corneal stroma develops edema and opacity due to a decrease in the corneal endothelial cell density, thus resulting in a drastic decrease in the eyesight. An effective treatment method is corneal transplantation alone. The greatest number of patients that undergo corneal transplantation suffer from bullous keratopathy (about 60%), and the estimated number of latent patients in our country is about 10,000. On the contrary, corneal transplantation is actually performed in 2500 cases annually and overwhelming shortage of cornea is widespread. Consequently, patients experiencing obstruction in daily living are forced to wait several years for the transplantation. Similarly cornea donors are extremely short in number all over the world except US,

In the meantime, conventional full-thickness corneal grafting for bullous keratopathy certainly improves eyesight from before surgery. However, the patients feel less satisfactory because strong irregular astigmatism, myopia and hyperopia occur after transplantation. Furthermore, the hospitalization period is long, actual stabilization of the eyesight often takes more than one year, and the eyesight 1.0 is restored less often. In recent years, as a new corneal transplantation, corneal endothelium transplantation (DSAEK) including partial grafting of parenchyma from the corneal endothelial layer of donor cornea has been tried. However, the thickness of graft which reaches 150 µm causes optical loss in the interface, and corrected eyesight cannot be sufficiently achieved (corrected eyesight about 0.5). Thus, expectation for the development of a new treatment method is extremely high.

The present inventors previously proposed a cornea reconstruction method as a method for developing a treatment method replacing conventional corneal transplantation, which includes preparing a laminate comprising a culture layer of human corneal endothelial cells obtained by culturing isolated and cultured human corneal endothelial cells on a transparent Type I collagen sheet, and transplanting the laminate into cornea from which corneal endothelium and Descemet's membrane have been removed (patent document 1). Also, it was found that a large amount of corneal endothelial cells can be cultured by cultivating corneal endothelial cells in a culture medium containing an ascorbic acid derivative (patent document 2). Thus, it is expected to supply a large number of corneal endothelial cell sheet for transplantation, which is at least as effective as corneal transplantation, from one cornea by combining these methods.

In addition, a production method of a corneal endothelial cell sheet using gelatin hydrogel as a support (patent document 3), and a corneal button comprising a biodegradable polymer-supported matrix which is absorbed by intraocular transplantation and an endothelial cell layer (patent document 4) are already known.

Incidentally, it is a common knowledge in seeding cells that a flat is generally used for seeding to achieve uniform seeding. When cells are seeded on a curved surface, there should be a special purpose such as formation of a cell aggregate for a mixed lymphocyte culture test and the like. Therefore, a flat sheet has heretofore been used exclusively to prepare a cultured cell sheet. For example, even when a cultured human cornea epithelial cell sheet or cultured human mouth cavity mucosal cell sheet is to be transplanted onto a surface of an eyeball having a curved surface similar to that of a corneal endothelium surface, cells are seeded on a circular, flat amniotic membrane, and clinical tests have been conducted without considering the curved surface of eyeball. This is because cultured cell sheet was considered to be usable without any problems due to the elasticity of the cells and cell carrier, even for transplantation into a site with concaves and convexes. In fact, no problem was perceived in the past even when a curved surface was not prepared.

Therefore, a similar practice was employed even for cultured corneal endothelial cell sheet. Particularly, since the transplantation area only needs to have a diameter of several mm to about 10 mm, the need for transplantation of a cultured corneal endothelial cell sheet having a curvature matching the corneal endothelium surface has not been considered at all. In view of the importance of uniform lamination of cells on a support, moreover, conception of seeding of cells on a sheet with a curved surface is far from achievable from the common knowledge.

While clinically-performed corneal endothelium transplantation is a treatment method including slicing a donor cornea and adhering of the graft onto a surface of corneal endothelium, which is a curved surface, it is performed without intentionally forming a curved surface.

### [Document List]

### [patent documents]

patent document 1: JP-A-2005-229869
patent document 2: WO 2011/096593
patent document 3: WO 2011/021706
patent document 4: WO 2007/047425

### SUMMARY OF THE INVENTION

### Problems to be Solved by the Invention

An object of the present invention is to develop a more superior corneal endothelial cell sheet that realizes recovery of visual function to that before decrease of eyesight, a short hospitalization period, and early recovery of eyesight after surgery, and provide an effective corneal endothelial cell transplantation therapy using same.

### Means of Solving the Problems

The present inventors have conducted intensive studies in an attempt to solve the aforementioned problems and found that, by seeding and cultivating human corneal endothelial cells on a cell support having a curvature fitting a corneal endothelium surface, a uniform corneal endothelial cell layer is unexpectedly formed on the support, and transplantation of the obtained corneal endothelial cell sheet having a curvature fitting a corneal endothelium surface into cornea without corneal endothelium affords a remarkably superior effect as compared to transplantation of a flat corneal endothelial cell sheet, which resulted in the completion of the present invention.

Accordingly, the present invention is as described below.
[1] A human corneal endothelial cell sheet having a curvature fitting a human corneal endothelial surface.
[2] The human corneal endothelial cell sheet of the above-mentioned [1], which is supported by a cell support having a curvature fitting a human corneal endothelial surface.
[3] The human corneal endothelial cell sheet of the above-mentioned [1] or [2], wherein the cell support is composed of collagen as a material.
[4] The human corneal endothelial cell sheet of the above-mentioned [3], wherein the sheet composed of collagen as a material is a gelatin sheet.
[5] The human corneal endothelial cell sheet of the above-mentioned [4], wherein the gelatin sheet has a thickness of 5 - 40 µm.
[6] The human corneal endothelial cell sheet of the above-mentioned [4] or [5], wherein the gelatin sheet is produced by the following steps:
   (1) a step of gelling a gelatin aqueous solution poured into a template having a curvature fitting a human corneal endothelial surface;
   (2) a step of forming a sheet-like gelatin by drying the gelled gelatin obtained in (1);
   (3) a step of obtaining a gelatin sheet by thermally crosslinking the sheet-like gelatin obtained in (2).
[7] The human corneal endothelial cell sheet of any of the above-mentioned [4] - [6], wherein the gelatin sheet is coated with laminin or atelocollagen.
[8] A production method of a human corneal endothelial cell sheet having a curvature fitting a human corneal endothelial surface, which comprises the following steps:
   (1) a step of gelling a gelatin aqueous solution poured into a template having a curvature fitting a human corneal endothelial surface;
   (2) a step of forming a sheet-like gelatin by drying the gelled gelatin obtained in (1);
   (3) a step of obtaining a gelatin sheet by thermally crosslinking the sheet-like gelatin obtained in (2);
   (4) a step of seeding human corneal endothelial cells on the gelatin sheet obtained in (3) to form a human corneal endothelial cell layer.
[9] The production method of the above-mentioned [8], wherein the gelatin aqueous solution has a concentration of 1 - 5 wt%.
[10] The production method of the above-mentioned [8] or [9], wherein the gelled gelatin has a thickness of 0.5 - 2 mm.
[11] The production method of any of the above-mentioned [8] - [10], wherein the gelatin sheet is coated with laminin or atelocollagen.
[12] The production method of any of the above-mentioned [8] - [11], wherein the gelatin sheet has a sheet thickness of 5 - 40 µm.
[13] The production method of any of the above-mentioned [8] - [12], wherein the template is made of Teflon (registered trade mark).

### Effect of the Invention

According to the present invention, a material for corneal endothelial tissue regenerative medicine, which is superior to conventional materials, can be provided.

### Brief Description of the Drawings

Fig. 1 shows top views and sectional views of the templates for producing a cell support and sectional views of the upper and lower templates laid on top of each other in one embodiment of the present invention.
Fig. 2 shows expression of functional marker proteins (A: ZO-1, B: Na⁺/K⁺ATPase) by human corneal endothelial cells 7 days after seeding on a curved gelatin sheet.
Fig. 3 shows cornea of Macaca fascicularis transplanted with a flat gelatin sheet and a curved gelatin sheet.
Fig. 4 shows a laminin-coated curved gelatin sheet (right) and a curved gelatin sheet free of coating (left) as observed under an inverted microscope.
Fig. 5 shows daily changes of the cornea thickness of Macaca fascicularis transplanted with a human corneal endothelial cell sheet.

### Description of Embodiments

The human corneal endothelial cell sheet of the present invention is characterized in that it has a curvature fitting a human corneal endothelial surface. The "curvature fitting a human corneal endothelial surface" means a curvature permitting direct and close adhesion of the human corneal endothelial cell sheet to the inner surface (transplanted surface) of human cornea from which corneal endothelium (or further Descemet's membrane) has been removed, when the human corneal endothelial cell sheet is transplanted into the human cornea inner surface. The human corneal endothelium surface is nearly spherical and varies depending on the individual. However, the radius of curvature fitting a human corneal endothelium surface is specifically about 6 - about 10 mm.

The "human corneal endothelial cell" in the present invention is a cobblestone-like cell present in the innermost layer of human eyeball cornea, or a cell obtained by separating and culturing the cobblestone-like cell. The human corneal endothelial cell may be harvested from human corneal endothelium, a human corneal endothelial cell produced by differentiation induction of undifferentiated cell (iPS cell, ES cell and the like), or an established human corneal endothelial cell. In consideration of the use of a corneal endothelial cell sheet obtained by applying the method of the present invention for corneal transplantation, it is desirably a primary cell harvested from human corneal endothelium or a passaged cell thereof.

Since cornea is an inaccessible antigen exempt from the recognition by the immune system, and the rejection does not always occur even when the HLA type does not match, a donor of human corneal endothelial cell does not entirely need to have an HLA type that matches the HLA type of the recipient. However, the cell is preferably collected from a human individual having not less than one locus, more preferably not less than 2 loci, further preferably not less than 3 loci, of HLA-A, HLA-B, HLA-DR and HLA-C, which are the same as those of the recipient.

A method of recovering human corneal endothelial cell from human corneal endothelium is not particularly limited, and those of ordinary skill in the art can appropriately select the method. For example, Descemet's membrane with human corneal endothelial cell attached thereto is harvested from a human sclerocorneal section, chopped, and cultivated in a medium containing collagenase under conditions of 5% CO₂, 37°C for 1 - 3 hr. Thereafter, fibroblast and the like are removed by centrifugal washing, and trypsin digestion is performed to give corneal endothelial cells as pellets.

In the above-mentioned method, collagenase A of Roche Ltd., collagenase type IA of Sigma Ltd., collagenase type I of Worthington Ltd. and the like can be used as collagenase, each of which is adjusted to 0.2% with a medium. As the medium, a DME medium containing 15% fetal calf serum (FCS) and 2 ng/mL basic fibroblast growth factor (bFGF) can be used.

The human corneal endothelial cell sheet of the present invention refers to a sheet-shaped structure obtained by culturing the above-mentioned human corneal endothelial cells, and may be any as long as it can be adhered to the back surface of corneal stroma and recover visual function after transplantation surgery. That is, it may be a sheet-shaped cell aggregate constituted of corneal endothelial cells alone, or a sheet-shaped structure formed by corneal endothelial cells and a support (biopolymer membrane) in combination. As a support (biopolymer membrane) to be added to form a sheet-shaped structure of corneal endothelial cells, or secreted by corneal endothelial cells to form a sheet-shaped structure, a biocompatible polymer and the like can be mentioned.

To have a curvature fitting a human corneal endothelial surface, the human corneal endothelial cell sheet desirably has a constitution wherein human corneal endothelial cells are supported by a cell support having a curvature fitting a human corneal endothelial surface. A cell support having a curvature fitting a human corneal endothelial surface has a contact lens-like thin layer sheet structure. The diameter of a circle (approximate circle) formed by the outer edge of the support sheet is appropriately selected according to the area of transplantation, which is generally about 5 - about 12 mm in consideration of the size of human cornea. The radius of curvature of the support sheet is generally about 6 - about 10 mm. While the thickness of the support sheet is appropriately about 5 - 100 µm, it is more preferably closer to the thickness of Descemet's membrane of human cornea. The thickness on transplantation (swelling) is preferably about 5 - about 40 µm, more preferably about 5 - about 25 µm.

The biopolymer constituting the cell support in the present invention is not particularly limited as long as it is a biocompatible polymer and can be formed to have a curvature fitting a human corneal endothelial surface. Examples thereof include a biopolymer composed of, as a material, a polymer complex made from one or more kinds of molecules selected from extracellular matrix molecules such as collagen, laminin, elastin, fibronectin, vitronectin, fibrinogen, thrombospondin, heparan sulfate, chondroitin sulfate and the like, RGDS, bFGF bonded to polycarbophil, EGF bonded to polycarbophil and the like. The cell support of the present invention may comprise an appropriate combination of one or more kinds of the above-mentioned biopolymer. The derivation of the biopolymer is not particularly limited, and not only biopolymers derived from human but those derived from swine, bovine, fish and the like can be used.

When collagen is used as the above-mentioned biopolymer, it can be purified from a collagen material before denaturation with acid or alkali in the process of gelatin production. As the collagen material, Type I collagen, Type II collagen, Type III collagen, Type IV collagen and the like are recited as examples, and these can also be used in combination. The derivation of collagen is not particularly limited. Commercially available collagen, for example, commercially available coating substrates for cell culture and the like can also be used. In addition, collagen prepared under various conditions such as heat denaturation, vitrification technique, peptide alteration and the like, can be used. Examples thereof include gelatin obtained by unwinding the triple-stranded helix structure by heat denaturation, vitrigel produced via a vitrification step, atelocollagen free of telopeptide and the like. These can also be used as a biopolymer using collagen as a material.

Preferable examples of the cell support in the present invention include the above-mentioned biopolymer using collagen as a material. Examples of the biopolymer using the above-mentioned collagen as a material include the above-mentioned gelatin, vitrigel, atelocollagen and the like, with particular preference given to gelatin. While gelatin is mainly produced from beef bones, beef skin, and pig skin as a material, skin and squama of fish such as salmon and the like are sometimes used as a material, and the derivation thereof is not particularly limited. A method of extracting and purifying gelatin from these materials is well known. In addition, commercially available gelatin can also be used.

Atelocollagen without immunoactivity can be particularly preferably used from the aspect of transplantation. Atelocollagen is obtained by separating from connective tissues such as skin, bone, blood vessel, tendon and the like of animals, by treating short fiber-like so-called insoluble collagen crosslinked between collagen molecules with protein separation enzymes such as pepsin and the like, alkali and the like to cleave and digest telopeptide which is present on both terminals of collagen molecules and involved in crosslinking. When atelocollagen derived from bovine is used, atelocollagen derived from skin without a risk of BSE infection is preferably used. As atelocollagen, atelocollagen powder and atelocollagen solution commercially available from Koken Co., Ltd. can be mentioned. Examples of the atelocollagen solution manufactured by Koken Co., Ltd. include IAC-30, IAC-50 (collagen acidic solution derived from bovine corium), and MEN-02, HAN-02, DME-02 (collagen neutral solution derived from bovine corium). Atelocollagen can also be used as a single component of a support, and other biopolymer supports can also be used by coating with atelocollagen.

When the above-mentioned biopolymer using collagen as a material is used as a cell support, a biopolymer support may also be coated with an extracellular matrix component other than atelocollagen and used. As such extracellular matrix component, an extracellular matrix molecule having affinity for collagen (gelatin) is preferable and, for example, extracellular matrix molecules such as laminin, elastin, fibronectin, vitronectin, fibrinogen and the like can be mentioned. Of these, laminin is preferably used to finely produce a corneal endothelial cell sheet having a curvature, and laminin-5 (laminin 332) is more preferably used. By coating a biopolymer support having a curvature with laminin, a more preferable human cornea cell sheet can be produced. While the coating method is not particularly limited, for example, a method including dissolving an extracellular matrix molecule such as laminin and the like at a suitable concentration in a solvent (e.g., PBS, acetic acid etc.), immersing a cell support in the solution and incubating same, a method including applying or spraying the solution on a cell support with a suitable tool and the like can be mentioned.

Examples of means for forming a biopolymer using collagen as a material into a cell support having a curvature fitting a human corneal endothelial surface include a method including pouring a solution of a biopolymer such as gelatin and the like into a template having a curvature fitting a human corneal endothelial surface, gelling same at a low temperature (e.g., 4°C), and drying same. While the concentration of a biopolymer solution when a biopolymer using collagen as a material such as gelatin and the like is used as a biopolymer is not particularly limited, it is preferably selected as appropriate from the range of about 1 - about 5 wt%.

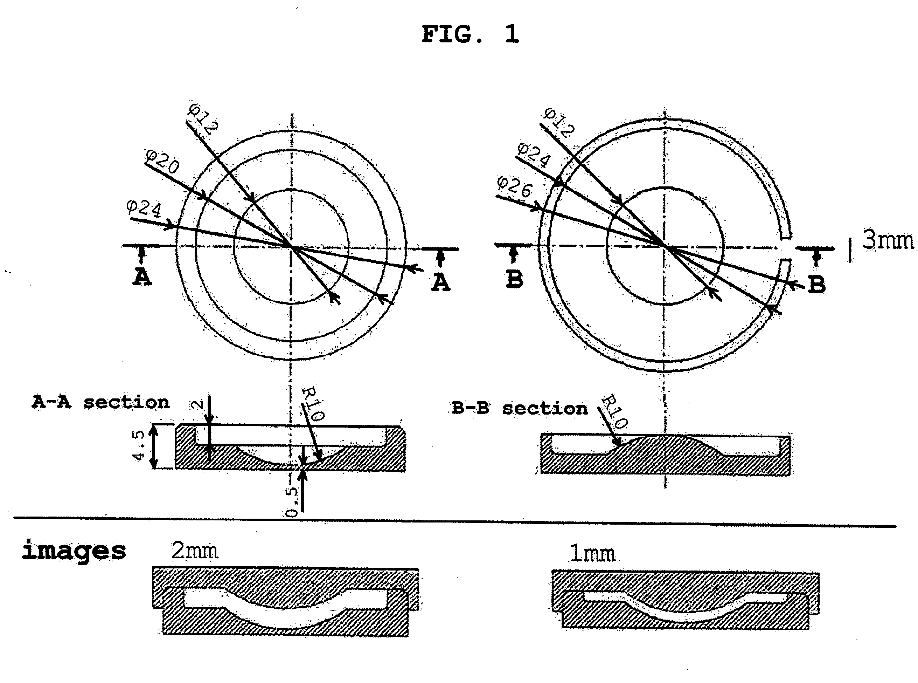

Examples of the template include, but are not limited to, acrylic, Teflon (registered trade mark), silicon templates and the like can be used. A biopolymer is poured into these templates, gelled and, after a drying step, formed into a sheet. The sheet after drying is extremely thin, and therefore, a Teflon (registered trade mark) template is preferably used since it permits easy removal of the sheet from the template without breakage. Using a template having a curvature fitting a human corneal endothelial surface, for example, as shown in Fig. 1, two upper and lower templates having concave and convex with the same radius of curvature are produced, a biopolymer solution is poured into the lower template, the upper template is placed as a lid, and the solution is solidified to give a desired cell support gel. When a cell support sheet of a biopolymer using collagen as a material is produced, the thickness of a support gel at the stage of formation using templates can be appropriately set to, for example, about 0.5 - about 2 mm. The gelled gelatin, collagen and the like are then dried to form a sheet-like shape. For drying, for example, air drying can be performed under aseptic conditions such as in a clean bench and the like.

The above-mentioned biopolymer is also preferably obtained by crosslinking a solution state or a shaped sheet. Crosslinking can be performed by a physical crosslinking method such as heating, UV or γ-ray radiation, or a chemical crosslinking method using a condensing agent such as watersoluble carbodiimide and the like. A cell support sheet having a desired thickness can be obtained by changing the crosslinking method, concentration of a condensing agent, treatment period, reaction temperature and the like.

In a particularly preferable embodiment of the present invention, a gelatin sheet is used as a cell support. To promote adhesion between a gelatin sheet and a human corneal endothelial cell, coating of a gelatin sheet with collagen (particularly, atelocollagen free of immunoactivity from the aspect of transplantation) is more preferable. As atelocollagen, those mentioned above can be used similarly. For atelocollagen coating of a gelatin sheet, for example, a gelatin sheet produced as mentioned above is immersed in about 1 - about 5 wt% atelocollagen solution, and incubating same at 20 - 50°C, preferably about 30 - 40°C, for about 0.5 - 3 hr.

The thus-obtained human corneal endothelial cell sheet composed of a cell support sheet having a curvature fitting a human corneal endothelial surface, and a human corneal endothelial cell laminated thereon can be produced, for example, by the following method.

### (1) preculture of human corneal endothelial cell

Human corneal endothelial cell harvested from human corneal endothelium as mentioned above can be cultured in a basal medium such as D-MEM, MEM and the like generally used for culture of animal cells (primary culture). For example, a medium (D-MEM etc.) having a low glucose concentration and supplemented with fetal bovine serum (FBS) 5 - 15%, growth factor and the like is preferable. The concentration of glucose contained in the medium is lower than general glucose concentration and is not more than 2.0 g/L, for example, 0.1-2.0 g/L, preferably 0.1 - 1.0 g/L. Examples of the growth factor include hepatocyte growth factor (HGF), epidermal growth factor (EGF), recombinant EGF (rEGF), fibroblast growth factor (FGF) and the like, and one or a combination of plural factors can be appropriately added to the medium. The concentration of these growth factors is generally 1 - 100 ng/mL, preferably 2 - 5 ng/mL. In addition to the above-mentioned medium composition, a preservative such as doxycycline and the like, and an antifungal agent such as Fungizone and the like can also be added as necessary. Furthermore, about 1.0 - 3.0%, preferably 1.0 - 2.0%, of hyaluronic acid (sodium hyaluronate) can also be added.

When a large amount of corneal endothelial cells are prepared from one human cornea, an ascorbic acid derivative only needs to be added to the medium in addition to the above-mentioned medium components. While ascorbic acid derivative is not particularly limited as long as it enhances proliferative capacity of the corneal endothelial cells, for example, ascorbic acid phosphoric acids such as ascorbic acid 2-phosphoric acid, ascorbic acid 2-diphosphoric acid, ascorbic acid 2-triphosphoric acid, ascorbic acid 2-polyphosphoric acid and the like; ascorbic acid esters such as ascorbic acid 2-phosphoric acid diester, ascorbic acid 2-phosphoric acid 6-palmitic acid, ascorbic acid 2-phosphoric acid 6-myristic acid, ascorbic acid 2-phosphoric acid 6-stearic acid, ascorbic acid 2-phosphoric acid 6-oleic acid, ascorbic acid 2-glucoside, ascorbic acid 2-glucoside 6-palmitic acid, ascorbic acid 2-glucoside 6-myristic acid, ascorbic acid 2-glucoside 6-stearic acid, ascorbic acid 2-glucoside 6-oleic acid, ascorbic acid 2-sulfuric acid and the like, L-ascorbic acid alkylester, L-ascorbic acid phosphate ester, L-ascorbic acid sulfuric acid ester and the like can be mentioned.

Salts with alkali metals such as sodium, potassium and the like, and salts with alkaline earth metals such as calcium, magnesium and the like, which are the salts of the above-mentioned ascorbic acid derivatives, are also encompassed in the ascorbic acid derivative. Of these, ascorbic acid 2-phosphoric acid is particularly preferable since it enhances the proliferative capacity of corneal endothelial cells.

The content of ascorbic acid derivative in the culture medium is not particularly limited as long as the proliferative capacity of the corneal endothelial cell is enhanced or a corneal endothelial cell sheet applicable to corneal transplantation can be obtained. Those of ordinary skill in the art can appropriately determine the content. However, a general index is normally 5 - 1,000 µg/mL, more preferably 20 - 100 µg/mL, from the aspect of mass culture of corneal endothelial cells.

Culture can be performed by attachment culture in a culture container (e.g., dish, petri dish, tissue culture dish, multidish, microplate, microwell plate, Multiplate, multiwell plate, chamber slide, petri dish, tube, tray, culture bag etc.) coated with a matrix such as Matrigel, collagen and the like.

The culture temperature is 35 - 38°C, preferably 37°C, and 90 - 100% moistened (preferably 100% moistened), 5 - 15% CO₂ (preferably 10% CO₂) incubator is used for culture. Culture can be performed until the cells become confluent (steady state for about 1 - 5 days).

The primary culture of human corneal endothelial cells is passaged at the time point when the cells become confluent. For example, cells are washed with PBS, dispersed in trypsin/EDTA, centrifuged, seeded in a culture container containing a medium similar to the above-mentioned at a cell density of 500 - 60,000 cells/cm², and cultivated under the above-mentioned culture conditions. A similar passage operation is repeated when the cells reach confluent. While the number of passages of the cells used for producing a human corneal endothelial cell sheet is not particularly limited, it is preferably about 1 - 5.

### (2) seeding of human corneal endothelial cell

The human corneal endothelial cells prepared as mentioned above are seeded on the aforementioned cell support sheet having a curvature fitting a human corneal endothelial surface and cultivated, whereby the human corneal endothelial cell sheet of the present invention is obtained. For cell seeding, a support sheet is left standing on the plate with a convex surface of the cell support sheet facing downward. For example, the Transwell plate (those having Snapwell inserts having 12 mm diameter membrane are preferable) of Corning Inc. wherein the membrane has been replaced with the cell support sheet can be used. In this case, the bottom surface of the cell support is fixed to be in contact with the culture dish. The thus-fixed cell support has fine concaves and convexes developed in the part in contact with the flat culture dish. A cell suspension is added dropwise on the thus-adjusted cell support sheet with a pipette and the like to seed the cells. Although the cells are non-uniformly adhered immediately after seeding due to the concaves and convexes of the cell support, a uniform human corneal endothelial cell layer (preferably a single layer) is surprisingly produced as cultivation proceeds.

When cells are seeded on a cell support sheet, a high quality cornea cell endothelial sheet for transplantation can be produced by seeding the cells at high density (seeding density: 2,000 - 8,000 cells/mm², preferably 4,000 - 6,000 cells/mm²) rather than growing the cells after seeding. Therefore, an ascorbic acid derivative is not always necessary at the time of seeding on the cell support sheet, and generally-used DME medium, MEM and the like can be used. For example, a medium (DME medium etc.) with a low glucose concentration and containing fetal calf serum (FCS), the aforementioned growth factor and the like can be used. For example, it is preferable from the aspects of affording functions (barrier function, pump function, cell adhesion ability) equivalent to those of uncultured corneal endothelial cells to culture cells in a DME medium containing 15% fetal calf serum and 2 ng/mL bFGF for not less than 1 to 2 weeks.

It is also possible to use a cell support sheet coated with an extracellular matrix components such as atelocollagen and laminin before seeding cells on the cell support sheet. Particularly, by coating with atelocollagen or laminin, cells do not accumulate at a particular position but surprisingly form a single layer structure more uniform than gelatin alone, and a human corneal endothelial cell sheet free of cell detachment and having more highly densely formed cells can be produced. The thus-produced preferable human corneal endothelial cell sheet has functions (barrier function, pump function, cell adhesion ability and the like) at least equivalent to those of uncultured corneal endothelial cells.

The thus-obtained human corneal endothelial cell sheet has a uniform human corneal endothelial cell layer (preferably a single layer) on a cell support, maintains functions as a corneal endothelial cell (barrier function, pump function, cell adhesion ability), which are equivalent to those of the cells in the living body, and has cell density at least equivalent to that of corneal endothelium of normal human eye (3,000 cells/mm²).

The thus-produced human corneal endothelial cell sheet can be transplanted into the backside of corneal stroma by a method known per se, for example, a method including removing the whole cornea, adhering a corneal endothelial cell sheet, and returning the cornea back to the original position (Hitani, K. et al., Mol. Vis. 2008 14: 1-9), a method including performing sclerocornea incision, wrapping a cultured corneal endothelial cell sheet in a silicon sheet and delivering same into the anterior chamber from the incision site with tweezers (Mimura, T. et al., Invest. Ophthalmol. Vis. Sci. 2004 45: 2992-7) and the like. However, these transplantation methods require high technique of the operator and accompany high invasion, and are defective since corneal endothelial cells are damaged during transplantation.

Therefore, to facilitate the operation for transplantation, it is desirably contained in a transplantation tool capable of maintaining a corneal endothelial cell in a viable state under an aseptic environment until use, and capable of noninvasively leading the sheet to the transplantation site in an eyeball during transplantation without, where possible, being affected by the technique of the operator. While such transplantation tool is not particularly limited, for example, the transplantation tool described in WO 2011/096593 can be preferably used.

The present invention is explained in more detail in the following by referring to Examples, which are not to be construed as limitative in any meaning.

### [Example 1]

### Preparation of 2% gelatin solution

The operation is desirably performed in a clean bench (safety cabinet). All containers and tools used had been sterilized or were of sterilized disposable type. BeMatrix gelatin LS-H powders (Nitta Gelatin Inc., 2.0 g) were weighted and placed in a bottle. Water (100 mL) for injection was added, and the mixture was shaken at room temperature for 1 hr, and dissolved by shaking in a 50°C thermostatic tank for 30 min. After cooling at room temperature, the mixture was preserved at 4°C.

### [Example 2]

### Preparation of curved gelatin sheet

2% gelatin preserved at 4°C was dissolved in a thermostatic tank at 37°C. A lid of the Teflon (registered trade mark) template having a curved surface, which was prepared by designing as shown in Fig. 1, was removed, the 2% gelatin solution (600 µL) was poured into the concave surface and spread all over the surface. The lid was placed back thereon, and the gelatin solution was cooled at 4°C for 1 day to allow for gelation. The lid of the template was slowly opened with tweezers, the gelled gelatin was dried in a clean bench (safety cabinet) for 2 days. After drying, the gelatin in a sheet shape was slowly detached from the template, and crosslinked in a vacuum drying apparatus at 140°C for 72 hr. As a comparison object, a flat gelatin sheet was prepared in the same manner using a flat template. The gelatin sheet after the crosslinking treatment was preserved at -30°C.

### [Example 3]

### Adhesion of gelatin sheet

Water (5 mL) for injection was poured into a 35 mm dish, and a curved gelatin sheet was allowed to swell in advance for one day. An insert with a membrane was taken out from Corning Inc. transwell (#3801), and the membrane was removed from the insert with tweezers. Water (5 mL) for injection was poured into a 35 mm dish, and the curved gelatin sheet swollen with water was floated thereon with the convex surface facing upward. A ring after removal of the membrane was placed under the curved gelatin sheet, the circumference of the ring and that of the sheet were matched and the water for injection was extracted. Wrinkles on the sheet were removed with tweezers, and the sheet was air-dried in a clean bench (safety cabinet). The ring and the curved sheet were immobilized with an O-ring, set in the transwell, and swollen with a growth medium.

### [Example 4]

### Preparation of laminin-coated gelatin sheet

Water for injection was removed from the transwell adhered with the curved gelatin sheet (swollen with water for injection), and PBS (160 µL) was added to the concave part of the curved sheet. Furthermore, a laminin solution (rLAMININ-5: Oriental Yeast Co., Ltd.) was added to PBS to a final concentration of 5 µg/mL and blended, and the mixture was incubated at 37°C for 2 hr. After washing twice with PBS (5 mL), a growth medium was added.

### [Example 5]

### Harvesting and primary culture of human corneal endothelial cells

A human sclerocorneal section was transferred into a 35 mm petri dish, and the endothelial surface was washed with a DME medium containing 15% fetal calf serum (FCS) and 2 ng/mL basic fibroblast growth factor (bFGF) (hereinafter to be described as basal medium).

Using fine tweezers, the corneal endothelium was detached in a sheet form together with the Descemet's membrane from the periphery toward the center of the inner surface of the cornea, and placed in a 35 mm petri dish. The Descemet's membrane section with the corneal endothelial cells attached thereto was further chopped into small pieces of 2 mm-square on the petri dish, recovered in a low adsorption centrifugal tube (manufactured by Sumitomo Bakelite Co., Ltd.), and incubated in a basal medium containing 0.2% collagenase (trade name: collagenase A, collagenase activity: >0.15 U/mg, manufactured by Roche Inc.) at 37°C, 5% CO₂ for 1 - 3 hr.

The collagenase-treated cells were diluted with the basal medium, repeatedly washed three times by centrifugation at 20 x g for 2 min, and the cells floating in the supernatant were removed. The cells were diluted with phosphate buffered saline (PBS), and similarly washed by centrifugation at 20 x g for 2 min. To the precipitated cell mass was added 0.5% trypsin/0.2% ethylenediaminetetraacetic acid (EDTA), and the mixture was incubated at 37°C, 5% CO₂ for 5 min. The basal medium was added, and the mixture was centrifuged at 500 x g for 5 min to give cell pellets. The obtained cell pellets were re-suspended in the basal medium containing 100 µg/mL ascorbic acid 2-phosphoric acid (manufactured by Wako Pure Chemical Industries, Ltd.), and the pellets were seeded on a dish produced by the following method and cultured in an incubator at 37°C, 5% CO₂ for 2 - 4 weeks by replacing the medium every 2 - 3 days.

### [Example 6]

### Preparation of atelocollagen-coated dish

2% Atelocollagen implant (manufactured by Koken Co., Ltd.) was diluted 400-fold with 10 mM acetic acid to prepare a 50 µg/mL atelocollagen implant solution. The solution (1 mL) was added to a 35 mm dish, left standing at 37°C for 1 hr and washed twice with PBS (2 mL) to prepare a dish coated with atelocollagen.

### [Example 7]

### Passage culture of human corneal endothelial cell

The primary cultured cells of the donor obtained in the above-mentioned Example 5 were each passage-cultured as follows.

The primary cultured cells were washed with PBS, and dispersed in 0.5% trypsin/0.2% EDTA. The basal medium was added thereto, and the mixture was centrifuged at 500 x g for 5 min, and suspended in the basal medium containing 100 µg/mL ascorbic acid 2-phosphoric acid. The cells were seeded on a dish coated with atelocollagen, which was produced by a method similar to that of the above-mentioned Example 5, at a cell density of 1,000 cells/cm², and cultured at 37°C, 5% CO₂. A similar passage operation was repeated at the time point when the cells reached confluent.

### [Example 8]

### Production of human corneal endothelial cell sheet (1)

The medium was removed from a transwell already adhered with a curved gelatin sheet (after medium swelling). In the same manner as in the above-mentioned Example 6, a 50 µg/mL atelocollagen implant solution was prepared, added at 5 mL/well, and left standing at 37°C for 1 hr. The atelocollagen implant solution was removed, the well was washed twice with PBS (5 mL/well), and the medium (5 mL) was added.

The passage-cultured cells were prepared to 1x10⁶ cells/mL, the medium was removed from the concave surface of the curved sheet, and a cell suspension (416 µL) was added. The mixture was cultured for 7 days under environment of 37°C, 5% CO₂.

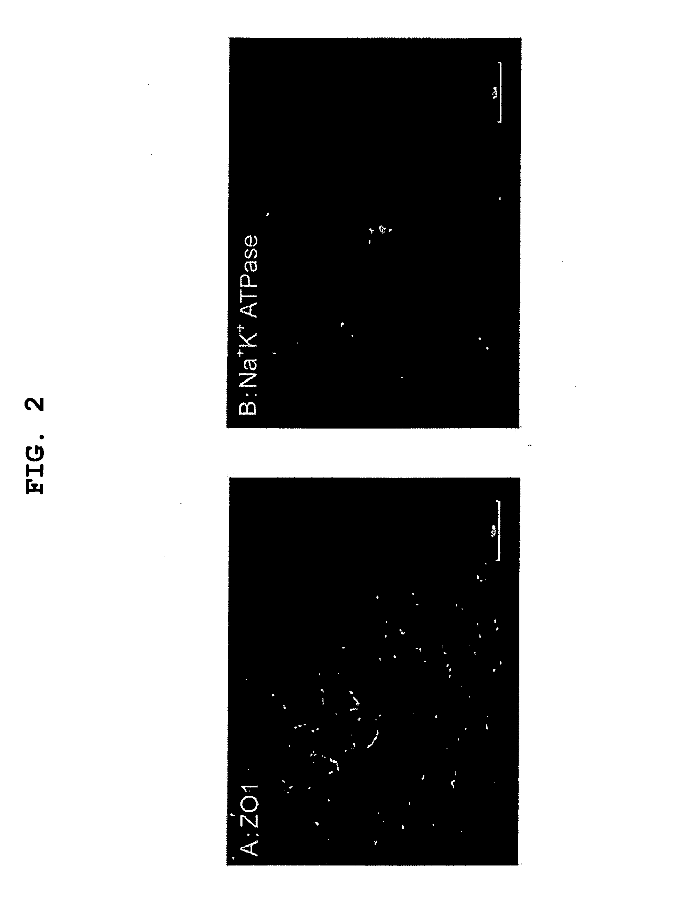

As a result, a single layer structure similar to endothelial cells in the living body was found. By immunostaining, expression of functional marker proteins (Fig. 2A: ZO-1, Fig. 2B: Na⁺/K⁺ ATPase) was also equivalent to that of endothelial cells in the living body. The density was calculated to be 3,000 cells/mm² or more.

### [Example 9]

### Production of human corneal endothelial cell sheet (2)

The growth medium was removed from the laminin-coated curved gelatin sheet obtained in Example 4, and a new growth medium (4 mL) was added. The passage-cultured cells were suspended in the basal medium to 1x10⁶ cells/mL, the medium was removed from the concave surface of the curvature sheet, and the cell suspension (416 µL) was added. The mixture was cultured under the environment of 37°C, 5% CO₂ for 7 days.

As a result, a single layer structure similar to that of the endothelial cells in the living body was found. Expression of functional marker proteins by immunostaining was also equivalent to that of endothelial cells in the living body and the above-mentioned (1).

### [Example 10]

### (1) Comparison of flat sheet and curved sheet

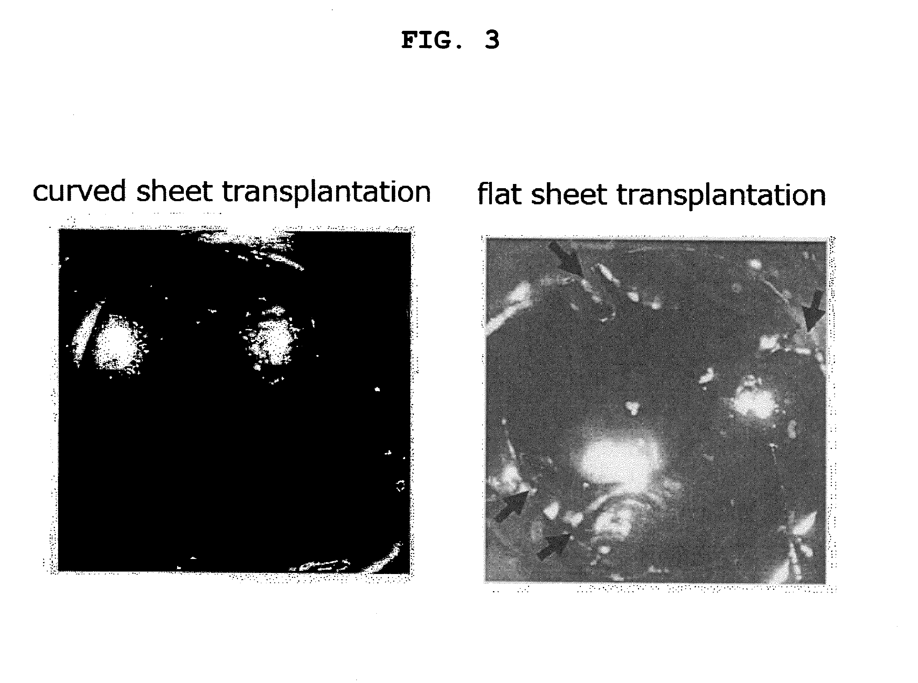

The curved and flat gelatin sheets prepared in Example 2 were placed on a 35 mm petri dish, immersed in distilled water for injection, and allowed to swell overnight. The swollen sheet was stained with Trypan Blue, cut out with 6 mm trephine, and filled in an injector for sheet transplantation. A cornea tunnel was formed in Macaca fascicularis with a scalpel, an injector was inserted in the anterior chamber, DSAEK tweezers were inserted from the side port produced on the opposite side of the injector insertion hole, and the sheet was drawn out from the injector. The sheet was adhered to the backside of cornea by injecting air, the incision was sutured with a 10-0 nylon suture thread, and the eyelid was sutured for completion. As shown in Fig. 3, when a flat gelatin sheet was transplanted as a conventional method, wrinkles were produced on the outer circumference of the sheet (wrinkles shown with arrows). In contrast, when a curved surface sheet, which is the product of the present invention, was transplanted, wrinkles were not produced on the sheet, and the sheet was closely adhered to the backside of cornea.

### (2) Observation of laminin-coated curved sheet

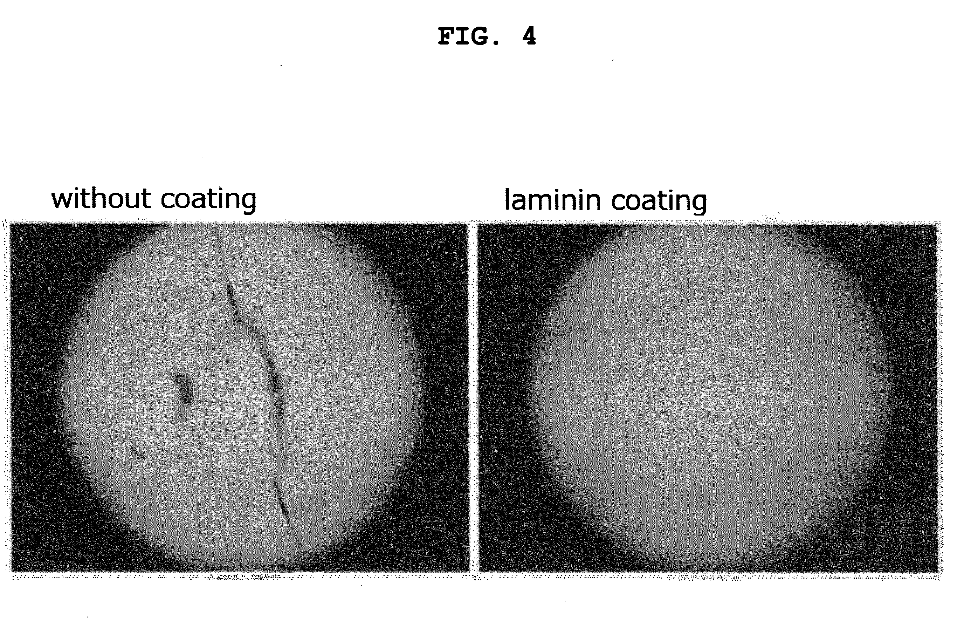

According to the method described in the above-mentioned Examples 4 and 9, the curved gelatin sheet was coated with laminin, and the cells were seeded on the sheet to produce a human corneal endothelial cell sheet. For comparison, the cells were also seeded on a curved gelatin sheet without coating. Each human corneal endothelial cell sheet was observed under an inverted microscope. As a result, when the cells were seeded on a simple gelatin sheet without coating, partial cell detachment was found depending on the lot (Fig. 4, left), but with laminin-5 coating, the cells did not detach at all (Fig. 4, right). The thus-obtained curved sheet was a uniform human corneal endothelial cell layer, and had a cell density of not less than 3,000 cell/mm², which is equivalent to corneal endothelium of normal human eye. In addition, the sheet was considered to maintain functions (barrier function, pump function and cell adhesion ability) as a corneal endothelial cell, which are at least equivalent to those of the cells in the living body.

This result shows that a human corneal endothelial cell sheet more suitable for transplantation can be obtained by using a laminin-coated gelatin sheet rather than a simple gelatin sheet.

### [Example 11]

### Transplantation of human corneal endothelial cell sheet

A corneal tunnel was formed in Macaca fascicularis with a scalpel, and the central endothelium (diameter about 8 mm) was rubbed with a 20G soft tapered needle. A viscoelastic substance was injected into the anterior chamber, and I/A washed. Trypan Blue solution diluted 5-fold with intraocular perfusion fluid was injected into the anterior chamber, I/A washed, and sufficient removal of the endothelial cells was confirmed. The cell sheet immobilized on the transwell of Example 7 was taken out by removing the whole transwell, and placed on a 35 mm petri dish. After staining with Trypan Blue, the sheet was cut out by 6 mm trephine. The sheet was washed with a serum-free medium, and the medium components were removed. A viscoelastic substance was applied on the sheet. A serum-free medium was filled again in the 35 mm petri dish, and the sheet was filled in the injector for sheet transplantation. The injector was inserted in the anterior chamber, DSAEK tweezers were inserted from the side port produced on the opposite side of the injector insertion hole, and the sheet was drawn out from the injector. The sheet was adhered to the backside of cornea by injecting air, the incision was sutured with a 10-0 nylon suture thread, and the eyelid was sutured for completion. Thereafter, the cornea thickness was measured every other week, and the state of edema was observed. As comparison control, a sheet transplantation-free group and a sheet alone transplantation group were used.

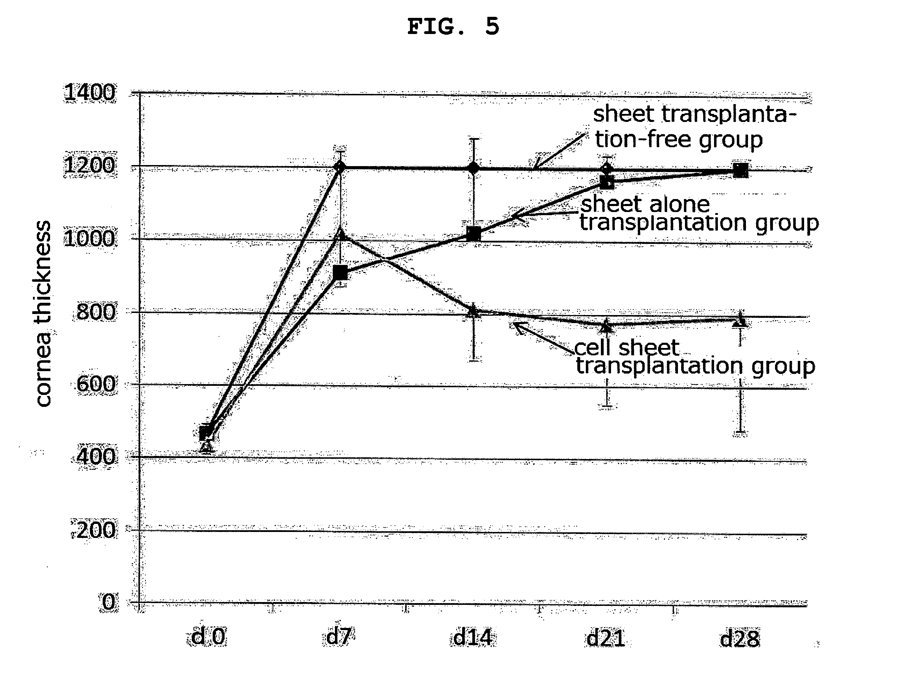

Profiles of the cornea thickness up to 4 weeks after surgery are shown in Fig. 5. As compared to the controls (sheet transplantation-free, sheet only), the human cell sheet transplantation group showed thinner cornea thickness, and the effectiveness thereof was clarified.

### Industrial Applicability

Since the human corneal endothelial cell sheet of the present invention has a curvature fitting a corneal endothelium surface, it affords a remarkably superior effect as compared to transplantation of a conventional flat corneal endothelial cell sheet, and therefore, the sheet is useful as a novel material for corneal endothelium tissue regenerative medicine.

This application is based on a patent application No. 2012-286050 filed in Japan (filing date: December 27, 2012), the contents of which are incorporated in full herein by reference.

## Claims

1. A human corneal endothelial cell sheet having a curvature fitting a human corneal endothelial surface.

2. The human corneal endothelial cell sheet according to claim 1, which is supported by a cell support having a curvature fitting a human corneal endothelial surface.

3. The human corneal endothelial cell sheet according to claim 1 or 2, wherein the cell support is composed of collagen as a material.

4. The human corneal endothelial cell sheet according to claim 3, wherein the sheet composed of collagen as a material is a gelatin sheet.

5. The human corneal endothelial cell sheet according to claim 4, wherein the gelatin sheet has a thickness of 5 - 40 µm.

6. The human corneal endothelial cell sheet according to claim 4 or 5, wherein the gelatin sheet is produced by the following steps:
(1) a step of gelling a gelatin aqueous solution poured into a template having a curvature fitting a human corneal endothelial surface;
(2) a step of forming a sheet-like gelatin by drying the gelled gelatin obtained in (1);
(3) a step of obtaining a gelatin sheet by thermally crosslinking the sheet-like gelatin obtained in (2).

7. The human corneal endothelial cell sheet according to any one of claims 4 to 6, wherein the gelatin sheet is coated with laminin or atelocollagen.

8. A production method of a human corneal endothelial cell sheet having a curvature fitting a human corneal endothelial surface, which comprises the following steps:
(1) a step of gelling a gelatin aqueous solution poured into a template having a curvature fitting a human corneal endothelial surface;
(2) a step of forming a sheet-like gelatin by drying the gelled gelatin obtained in (1);
(3) a step of obtaining a gelatin sheet by thermally crosslinking the sheet-like gelatin obtained in (2);
(4) a step of seeding human corneal endothelial cells on the gelatin sheet obtained in (3) to form a human corneal endothelial cell layer.

9. The production method according to claim 8, wherein the gelatin aqueous solution has a concentration of 1 - 5 wt%.

10. The production method according to claim 8 or 9, wherein the gelled gelatin has a thickness of 0.5 - 2 mm.

11. The production method according to any one of claims 8 to 10, wherein the gelatin sheet is coated with laminin or atelocollagen.

12. The production method according to any one of claims 8 to 11, wherein the gelatin sheet has a sheet thickness of 5 - 40 µm.

13. The production method according to any one of claims 8 to 12, wherein the template is made of Teflon (registered trade mark).
